# EUROPEAN PATENT SPECIFICATION

(11) **EP 3 344 328 B1**
(45) Date of publication and mention of the grant of the patent: **18.03.2026**
(21) Application number: 16842785.4
(22) Date of filing: 30.08.2016
(51) Int. Cl.: A61N 1/00

(54) **COCHLEAR IMPLANT FOR PATIENTS WITH RESIDUAL HEARING**
COCHLEA-IMPLANTAT FÜR PATIENTEN MIT RESTGEHÖR
IMPLANT COCHLÉAIRE POUR PATIENTS AYANT UNE AUDITION RÉSIDUELLE

(30) Priority: 01.09.2015 US 201562212642 P; 01.09.2015 US 201562212643 P
(43) Date of publication of application: 11.07.2018
(73) Proprietor: MED-EL Elektromedizinische Geräte GmbH, 6020 Innsbruck (AT)
(72) Inventor: POLAK, Marek, 6020 Innsbruck (AT)
(74) Representative: Lucke, Andreas
(86) International application number: PCT/US2016/049387
(87) International publication number: WO 2017/040466

(56) References cited:
- WO-A1-2014/123890
- US-A1- 2011 066 210
- US-A1- 2013 245 740
- US-A1- 2014 005 746
- US-A1- 2014 324 122

## Description

### FIELD OF THE INVENTION

The present invention relates to hybrid electric acoustic (EAS) hearing systems.

### BACKGROUND ART

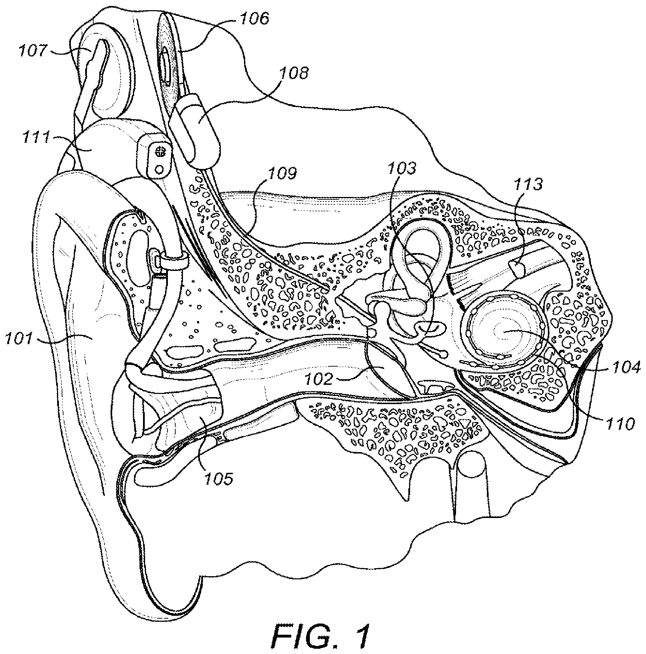

A normal ear transmits sounds as shown in Figure 1 through the outer ear **101** to the tympanic membrane **102,** which moves the bones of the middle ear **103** (malleus, incus, and stapes) that vibrate the oval window and round window openings of the cochlea **104.** The cochlea **104** is a long narrow duct wound spirally about its axis for approximately two and a half turns. It includes an upper channel known as the scala vestibuli and a lower channel known as the scala tympani, which are connected by the cochlear duct. The cochlea **104** forms an upright spiraling cone with a center called the modiolar where the spiral ganglion cells of the acoustic nerve **113** reside. In response to received sounds transmitted by the middle ear **103,** the fluid-filled cochlea **104** functions as a transducer to generate electric pulses which are transmitted to the cochlear nerve **113,** and ultimately to the brain.

Hearing is impaired when there are problems in the ability to transduce external sounds into meaningful action potentials along the neural substrate of the cochlea **104.** To improve impaired hearing, hearing prostheses have been developed. For example, when the impairment is related to operation of the middle ear **103,** a conventional hearing aid may be used to provide mechanical stimulation to the auditory system in the form of amplified sound. Or when the impairment is associated with the cochlea **104,** a cochlear implant with an implanted stimulation electrode can electrically stimulate auditory nerve tissue with small currents delivered by multiple electrode contacts distributed along the electrode.

In some patients with some residual hearing in the lower acoustic frequencies, a conventional hearing aid and a cochlear implant can be combined together in a hybrid Electric Acoustic Stimulation (EAS) system. The hearing aid acoustically amplifies lower acoustic frequencies perceived by human ear, while the cochlear implant electrically stimulates the middle and high frequencies. *See* von Ilberg et al, Electric-Acoustic Stimulation of the Auditory System, ORL 61:334-340; Skarzynski et al, Preservation of Low Frequency Hearing in Partial Deafness Cochlear Implantation (PDCI) Using the Round Window Surgical Approach, Acta OtoLaryngol 2007;127:41-48; Gantz & Turner, Combining Acoustic and Electrical Speech Processing: Iowa/Nucleus Hybrid Implant, Acta Otolaryngol 2004;124:344-347; Gstöttner et al., Hearing Preservation in Cochlear Implantation for Electric Acoustic Stimulation, Acta Otolaryngol 2004; 124 348-352.

Figure 1 also shows some components of a typical EAS system which includes an external microphone that provides an acoustic signal input to an external signal processor **111** where two different signal processing paths are developed. An upper acoustic frequency range communications signal containing middle and high frequency range acoustic is converted into a digital data format, such as a sequence of data frames, for transmission via a transmitter coil **107** over a corresponding implanted receiver coil **106** into the electric implant **108** (a typical cochlear implant system). Besides receiving the processed acoustic information, the electric implant **108** also performs additional signal processing such as error correction, pulse formation, etc., and produces an electric stimulation pattern (based on the extracted acoustic information) that is sent through an electrode lead **109** to an implanted electrode array **110.** Typically, this electrode array **110** includes multiple electrode contacts on its outer surface that provide selective electric stimulation of the cochlea **104.** The external signal processor **111** also creates a lower acoustic frequency range communications signal to a conventional hearing aid **105** in the ear canal which acoustically stimulates the tympanic membrane **102,** and in turn the middle ear **103** and cochlea **104.**

In some coding strategies, stimulation pulses are applied at a constant rate across all electrode channels, whereas in other coding strategies, stimulation pulses are applied at a channel-specific rate. Various specific signal processing schemes can be implemented to produce the electrical stimulation signals. Signal processing approaches that are well-known in the field of cochlear implants include continuous interleaved sampling (CIS), channel specific sampling sequences (CSSS) (as described in U.S. Patent No. 6,348,070), spectral peak (SPEAK), and compressed analog (CA) processing.

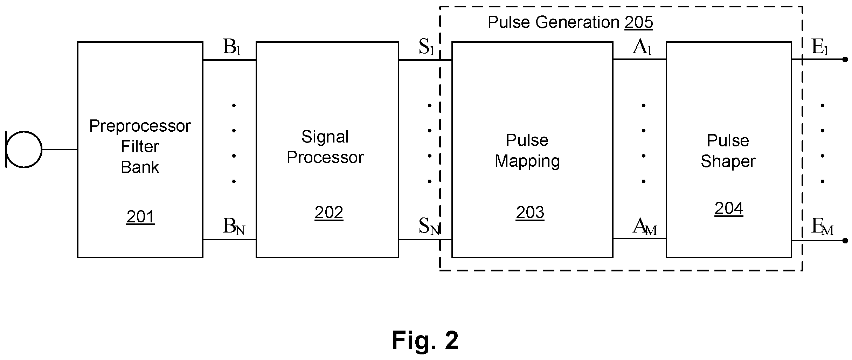

Figure 2 shows the major functional blocks in a typical cochlear implant signal processing system wherein band pass signals are processed and coding to generate electrode stimulation signals to stimulation electrodes in an implanted cochlear implant electrode array. For example, commercially available Digital Signal Processors (DSP) can be used to perform speech processing according to a 12-channel CIS approach. The initial acoustic audio signal input is produced by one or more sensing microphones, which may be omnidirectional and/or directional. Preprocessor Filter Bank **201** pre-processes the initial acoustic audio signal with a bank of multiple band pass filters, each of which is associated with a specific band of audio frequencies-for example, a digital filter bank having 12 digital Butterworth band pass filters of 6th order, Infinite Impulse Response (IIR) type-so that the acoustic audio signal is filtered into some *M* band pass signals, *B*₁ to *B_{M}* where each signal corresponds to the band of frequencies for one of the band pass filters. Each output of the CIS band pass filters can roughly be regarded as a sinusoid at the center frequency of the band pass filter which is modulated by the envelope signal. This is due to the quality factor (Q ≈ 3) of the filters. In case of a voiced speech segment, this envelope is approximately periodic, and the repetition rate is equal to the pitch frequency. Alternatively and without limitation, the Preprocessor Filter Bank **201** may be implemented based on use of a fast Fourier transform (FFT) or a short-time Fourier transform (STFT). Based on the tonotopic organization of the cochlea, each electrode contact in the scala tympani often is associated with a specific band pass filter of the external filter bank.

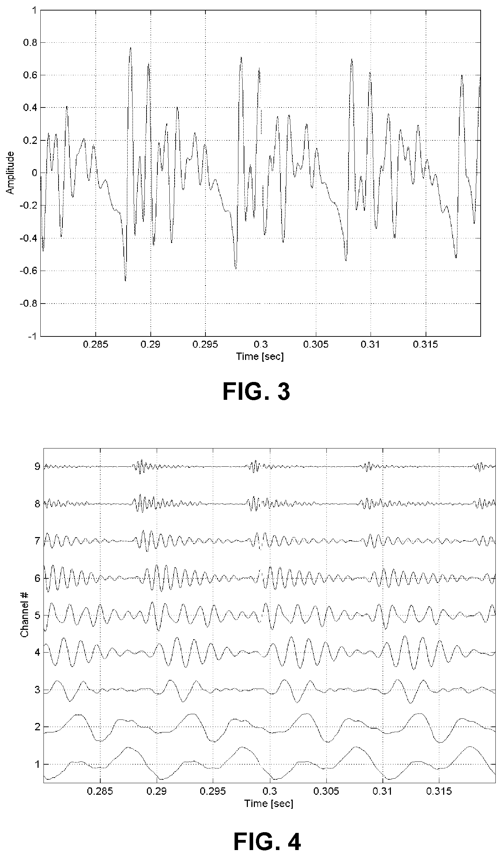

Figure 3 shows an example of a short time period of an audio speech signal from a microphone, and Figure 4 shows an acoustic microphone signal decomposed by band-pass filtering by a bank of filters into a set of signals. An example of pseudocode for an infinite impulse response (IIR) filter bank based on a direct form II transposed structure is given by Fontaine et al., Brian Hears: Online Auditory Processing Using Vectorization Over Channels, Frontiers in Neuroinformatics, 2011:

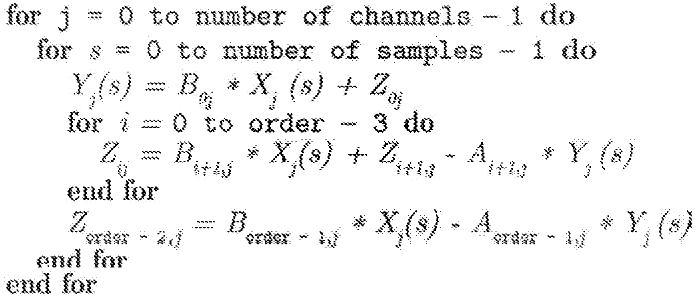

The band pass signals *B*₁ to *B_{M}* (which can also be thought of as frequency channels) are input to a Signal Processor **202** which extracts signal specific stimulation information-e.g., envelope information, phase information, timing of requested stimulation events, etc.-into a set of *N* stimulation channel signals *S*₁ to *S*_{N} that represent electrode specific requested stimulation events. For example, channel specific sampling sequences (CSSS) may be used as described in U.S. Patent 6,594,525. For example, the envelope extraction may be performed using 12 rectifiers and 12 digital Butterworth low pass filters of 2nd order, IIR-type.

A Pulse Generator **205** includes a Pulse Mapping Module **203** that applies a non-linear mapping function (typically logarithmic) to the amplitude of each band-pass envelope. This mapping function-for example, using instantaneous nonlinear compression of the envelope signal (map law)-typically is adapted to the needs of the individual cochlear implant user during fitting of the implant in order to achieve natural loudness growth. This may be in the specific form of functions that are applied to each requested stimulation event signal *S*₁ to *S*_{N} that reflect patient-specific perceptual characteristics to produce a set of electrode stimulation signals *A*₁ to *A*_{M} that provide an optimal electric representation of the acoustic signal. A logarithmic function with a form-factor *C* typically may be applied as a loudness mapping function, which typically is identical across all the band pass analysis channels. In different systems, different specific loudness mapping functions other than a logarithmic function may be used, with just one identical function is applied to all channels or one individual function for each channel to produce the electrode stimulation signals *A*₁ to *A*_{M} outputs from the Pulse Mapping Module **203.**

The Pulse Generator **205** also includes a Pulse Shaper **204** that develops the set of electrode stimulation signals *A*₁ to *A*_{M} into a set of output electrode pulses *E*₁ to *E*_{M} for the electrode contacts in the implanted electrode array which stimulate the adjacent nerve tissue. The electrode stimulation signals *A*₁ to *A*_{M} may be symmetrical biphasic current pulses with amplitudes that are directly obtained from the compressed envelope signals.

In the specific case of a CIS system, the stimulation pulses are applied in a strictly non-overlapping sequence. Thus, as a typical CIS-feature, only one electrode channel is active at a time and the overall stimulation rate is comparatively high. For example, assuming an overall stimulation rate of 18 kpps and a 12 channel filter bank, the stimulation rate per channel is 1.5 kpps. Such a stimulation rate per channel usually is sufficient for adequate temporal representation of the envelope signal. The maximum overall stimulation rate is limited by the minimum phase duration per pulse. The phase duration cannot be arbitrarily short because, the shorter the pulses, the higher the current amplitudes have to be to elicit action potentials in neurons, and current amplitudes are limited for various practical reasons. For an overall stimulation rate of 18 kpps, the phase duration is 27 µs, which is near the lower limit.

In the CIS strategy, the signal processor only uses the band pass signal envelopes for further processing, i.e., they contain the entire stimulation information. For each electrode channel, the signal envelope is represented as a sequence of biphasic pulses at a constant repetition rate. A characteristic feature of CIS is that the stimulation rate is equal for all electrode channels and there is no relation to the center frequencies of the individual channels. It is intended that the pulse repetition rate is not a temporal cue for the patient (i.e., it should be sufficiently high so that the patient does not perceive tones with a frequency equal to the pulse repetition rate). The pulse repetition rate is usually chosen at greater than twice the bandwidth of the envelope signals (based on the Nyquist theorem).

Another cochlear implant stimulation strategy that does transmit fine time structure information is the Fine Structure Processing (FSP) strategy by Med-El. Zero crossings of the band pass filtered time signals are tracked, and at each negative to positive zero crossing, a Channel Specific Sampling Sequence (CSSS) is started. Typically CSSS sequences are only applied on the first one or two most apical electrode channels, covering the frequency range up to 200 or 330 Hz. The FSP arrangement is described further in Hochmair I, Nopp P, Jolly C, Schmidt M, Schößer H, Garnham C, Anderson I, MED-EL Cochlear Implants: State of the Art and a Glimpse into the Future, Trends in Amplification, vol. 10, 201-219, 2006.

Many cochlear implant coding strategies use what is referred to as an N-of-M approach where only some number *n* electrode channels with the greatest amplitude are stimulated in a given sampling time frame. If, for a given time frame, the amplitude of a specific electrode channel remains higher than the amplitudes of other channels, then that channel will be selected for the whole time frame. Subsequently, the number of electrode channels that are available for coding information is reduced by one, which results in a clustering of stimulation pulses. Thus, fewer electrode channels are available for coding important temporal and spectral properties of the sound signal such as speech onset.

One method to reduce the spectral clustering of stimulation per time frame is the MP3000^{™} coding strategy by Cochlear Ltd, which uses a spectral masking model on the electrode channels. Another method that inherently enhances coding of speech onsets is the ClearVoice^{™} coding strategy used by Advanced Bionics Corp, which selects electrode channels having a high signal to noise ratio. U.S. Patent Publication 2005/0203589 describes how to organize electrode channels into two or more groups per time frame. The decision which electrode channels to select is based on the amplitude of the signal envelopes.

In addition to the specific processing and coding approaches discussed above, different specific pulse stimulation modes are possible to deliver the stimulation pulses with specific electrodes *-i.e.* mono-polar, bi-polar, tri-polar, multi-polar, and phased-array stimulation. And there also are different stimulation pulse shapes-*i*.*e*. biphasic, symmetric triphasic, asymmetric triphasic pulses, or asymmetric pulse shapes. These various pulse stimulation modes and pulse shapes each provide different benefits; for example, higher tonotopic selectivity, smaller electrical thresholds, higher electric dynamic range, less unwanted side-effects such as facial nerve stimulation, etc. But some stimulation arrangements are quite power consuming, especially when neighboring electrodes are used as current sinks. Up to 10 dB more charge might be required than with simple mono-polar stimulation concepts (if the power-consuming pulse shapes or stimulation modes are used continuously).

It is well-known in the field that electric stimulation at different locations within the cochlea produce different frequency percepts. The underlying mechanism in normal acoustic hearing is referred to as the tonotopic principle. In cochlear implant users, the tonotopic organization of the cochlea has been extensively investigated; for example, see Vermeire et al., Neural tonotopy in cochlear implants: An evaluation in unilateral cochlear implant patients with unilateral deafness and tinnitus, Hear Res, 245(1-2), 2008 Sep 12 p. 98-106; and Schatzer et al., Electric-acoustic pitch comparisons in single-sided-deaf cochlear implant users: Frequency-place functions and rate pitch, Hear Res, 309, 2014 Mar, p. 26-35. According to the Greenwood scale, 360° of electrode insertion in the tonotopically organized cochlea covers the acoustic frequency region from 1 kHz and higher. *See* Greenwood, A Cochlear Frequency-Position Function For Several Species - 29 Years Later, J Acoustic Soc Am, 1990;87(6) 2592-2605.

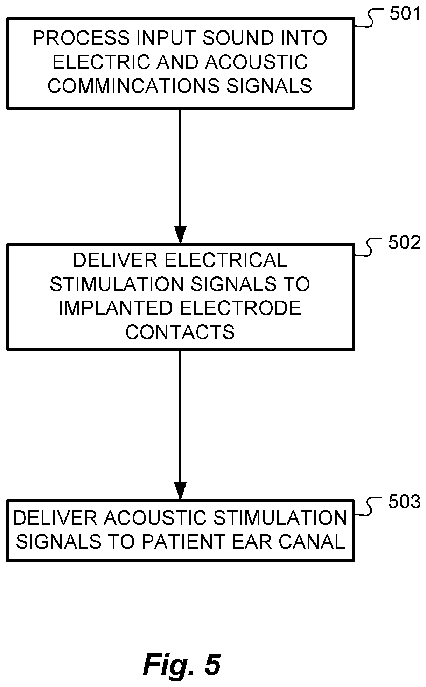

In a normal hearing ear, one frequency component consecutively stimulates multiple neural populations. This phenomenon was described as the "travelling wave" as shown in Figure 5 from Von Békésy, Georg. *Experiments in hearing.* Ed. Ernest Glen Wever. Vol. 8. New York: McGraw-Hill, 1960. That is, in response to a pure tone, the basilar membrane resonates in a travelling wave (the ascending numbers within Fig. 5) which gradually grows in amplitude (the dashed lines in Fig. 5) as it moves along the cochlear duct from the stapes (base) toward the helicotrema (apex).

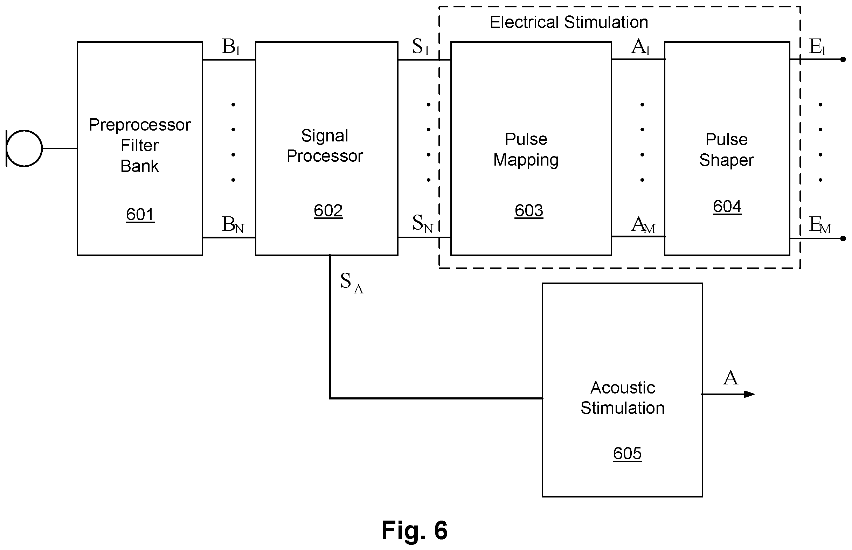

One quality of the travelling wave that is partly reflected in modern cochlear implant systems is that each frequency component reaches a peak amplitude at a specific spot within the cochlea (the tonotopic principle discussed above). These spectro-temporal properties can also be observed in the activity of cat's cochlear nerve fibres shown in Figure 6 from Secker Walker et al, Time domain analysis of auditory nerve fiber firing rates, J Acoust Soc Am , 88(3), 1990, p. 1427-1436. Figure 6 shows neural activity in the cochlear nerve over time at nerve fibres with different characteristic frequencies in response to synthetic vowels. One dominant frequency component in the synthetic vowel stimuli is the fundamental frequency (F0), which in Fig. 6 can be clearly identified as a regular pattern starting at high frequencies and ending several milliseconds later at low frequencies. The black curve in the shaded box in Fig. 6 indicates the frequency-specific time delays or the neural responses. Higher frequency components also can be observed between the F0 structures; for example, harmonics that are visible between 1800 and 1000 Hz. Similar to the F0 structures, they start at high frequency fibers and end some milliseconds later at low frequency fibers. This spectro-temporal excitation behaviour is not currently explicitly implemented in cochlear implant systems.

Loeb G., Are cochlear implant patients suffering from perceptual dissonance? Ear Hear, 26, 2005, p. 435-450 describes that phase-locking occurs over a substantial length of the cochlea. Furthermore, the action potentials exhibit a coherent spatial gradient with the steepest and most rapidly changing gradient of the phase occurring next to the place of the resonant frequency. At this point, the travelling wave starts to significantly slow down and dissipates. The phase gradient is believed to substantially contribute to pitch perception, especially in loud situations where harmonics are not resolved.

Existing coding approaches take into account some of the temporal properties of the acoustic signal. CIS determines frequency-specific envelopes which inherently contain a certain amount of information about individual low frequency components such as the fundamental frequency. More advanced approaches for calculating band specific envelopes also have been described; for example, U.S. Patent Publication 2006/0235486. The latter and CIS both sample the band pass envelopes with fixed rate stimulation pulses to resemble rudimentary properties of the basilar membrane movement. Other advanced systems as described in U.S. Patent Publication 2011/0230934 explicitly extract temporal characteristics of a band pass signal by identifying phase characteristics such as zero crossings. The described system triggers channel-specific sequences of stimulation pulses at each detected zero crossing. Each of the foregoing arrangements attributes certain frequency components to certain stimulation places. U.S. Patent Publication 2011/0230934 also explicitly takes into account the timing of certain frequency components.

Vocoder-based cochlear implant stimulation arrangements such as CIS and N-of-M do not take into account the travelling wave properties of normal acoustic hearing. The acoustic signal is analysed by filter banks or FFT and assigned either to single intracochlear electrodes, or to simultaneous stimulation of multiple adjacent electrodes. While filter banks can mimic the latencies of single frequency components at the place of stimulation, they are not able to mimic other aspects of the travelling wave behaviour such as the spectro-temporal distribution of this component to neighbouring stimulation sites, starting at a more basal site with low amplitude and ending at a more apical stimulation site with a maximum of stimulation at a site in between. An FFT, also used for mimicking the tonotopic principle in a cochlear implant is no better able to replicate the general latency differences between the frequency components (at the place of stimulation) nor does it provide the spectro-temporal behaviour described above.

In those patients receiving hearing implants who retain significant post-surgical residual natural hearing, typically, the residual hearing is in the lower frequency bands, which tonotopically corresponds to the deepest locations within the cochlea. In some cases, an apical portion of the implanted electrode array reaches far enough in to the cochlea to enter into the regions with residual hearing. In such cases, it has been suggested that the most apical electrode channels be turned off. For example, U.S. 2013/0116746 suggests that the electrode array be inserted only to a shallow depth in the scala tympani which typically is a region without residual hearing. Or if the most apical contacts of the electrode array actually are located in the acoustically perceivable region of the patient, to intentionally introduce a frequency gap between the acoustically perceivable frequencies and the electrically perceivable frequencies by turning off one or more of the most apical electrode contacts to create a (small) region without any hearing stimulation, either acoustic or electric.

U.S. Patent 8,000,798 suggests an electrode array that after insertion has a subset of electrode contacts beyond a first basal turn of the cochlea, and activating one or more of the contacts in this subset while simultaneously allowing natural acoustic hearing to occur in one or more locations beyond the first basal turn. But it is apparent from the description that this patent describes "a relatively very thin and short electrode array that is insertable into the basal region of the cochlea and past the first turn thereof."

WO 00/69513 suggests a hybrid cochlear stimulation arrangement (i.e., combined electrical stimulation together with acoustic stimulation of residual hearing) that uses an electrode array which is no longer than 8mm-shorter than the distance from the cochleostomy or round window membrane to the first basal turn.

All these existing measures are based on current beliefs that electrically stimulated hearing is different from natural acoustic hearing, and that electric stimulation interacts with acoustic hearing.
WO 2014/123890 discloses a system and method of fitting an Electric Acoustic Stimulation (EAS) system of a patient that has binaural hearing. The patient has an ipsilateral ear and a contralateral ear opposite the ipsilateral ear, with the EAS system associated with the ipsilateral ear of the patient. Stimulation parameters of the EAS system are developed unilaterally while taking into account hearing abilities of the contralateral ear. The EAS system is configured based on the developed stimulation parameters. In case of overlap between the usable acoustic residual hearing and frequency range covered by the electrode insertion according to the Greenwood scale, the most apical electrodes can be turned off in order to allow purely acoustic stimulation in the region of usable acoustic hearing.
US 2014/0324122 discloses a method of activating at least two electrodes in a multichannel electrode array using channel specific sampling sequences. A channel specific sampling sequence is defined for each electrode, the sequence having a particular duration, pulse amplitude distribution, and number of pulses. A weighting factor is applied to the channel specific sampling sequence. Each electrode in the multichannel electrode array is then simultaneously activated using sign-correlated pulses, the sign-correlated pulses based on parameters of spatial channel interaction reflecting geometric overlapping of electrical fields from each electrode, non-linear compression, and each electrode's weighted channel specific sampling sequence.

### SUMMARY OF THE INVENTION

The invention is defined in claim 1. A hearing signal processor is configured to process an input sound signal and generate: i. an electrical communications signal representative of an upper range of sound frequencies present in the input sound signal, and ii. an acoustic communications signal representative of a lower range of sound frequencies present in the input sound signal. An implanted electrical stimulation subsystem includes an electrode array having a plurality of electrode contacts implanted in a patient cochlea with one or more electrode contacts in an acoustically perceivable cochlear region retaining residual natural hearing. The electrical stimulation subsystem is configured to receive the electrical communications signal from the hearing signal processor and deliver corresponding electrical stimulation signals to the electrode contacts for electrical stimulation of adjacent neural tissue. An external acoustic stimulation subsystem is configured to receive the acoustic communications signal from the hearing signal processor and deliver corresponding amplified acoustic stimulation signals to the ear canal of the patient. The hearing signal processor is configured to overlap the upper range and the lower range and coordinate the electrical stimulation signals and the amplified acoustic stimulation signals for simultaneous delivery to the acoustically perceivable cochlear region.

In further specific embodiments, the electrical stimulation signals may be frequency specific sampling sequence (FSSS) and/or channel specific sampling sequence (CSSS) signals. The electrical stimulation signals also may be a patient-specific, frequency-specific function of stimulation location, rate, and level. The electrical stimulation signals for the one or more electrode contacts include fine structure information. And the one or more electrode contacts in the acoustically perceivable cochlear region may be implanted at least 24 mm into the patient cochlea.

### BRIEF DESCRIPTION OF THE DRAWINGS

Figure 1 shows a section view of a human ear with a typical cochlear implant system designed to deliver electrical stimulation to the inner ear.
Figure 2 shows various functional blocks in a continuous interleaved sampling (CIS) processing system.
Figure 3 shows an example of a short time period of an audio speech signal from a microphone.
Figure 4 shows an acoustic microphone signal decomposed by band-pass filtering by a bank of filters into a set of band pass signals.
Figure 5 shows various logical blocks in a process of combined electrical and acoustic stimulation according to an embodiment of the present invention.
Figure 6 shows various functional blocks in a combined electrical and acoustic stimulation system according to an embodiment of the present invention.
Figure 7 shows various logical blocks in a process of fitting a combined electrical and acoustic stimulation system according to an embodiment of the present invention.

### DETAILED DESCRIPTION OF SPECIFIC EMBODIMENTS

Frequency specific sampling sequence (FSSS) arrangements such as those descried in co-pending U.S. Provisional Patent Application 62/212,643, filed September 1, 2015, and rate-location matched stimulation arrangements such as those descried in co-pending U.S. Provisional Patent Application 62/212,642, filed September 1, 2015 and in from German Patent Application DE 102015104614, filed March 26, 2015, can be particularly useful for patients with residual natural acoustic hearing who receive an EAS hybrid system. In such cases, a deeply inserted electrode array can be used which is inserted with the apical portion inside the acoustically functioning interior of the cochlea. This acoustically functioning portion of the cochlea that contains the electrode array is referred to as the "A region." Similarly, "deep insertion" in this context refers to electrode arrays having a length, for example, of 24-32 mm, where the latter length corresponds to complete cochlear coverage. Simultaneous acoustic and electric stimulation in the A region means that nerve signals are elicited in response to natural acoustic stimulation and also in response to the application of electrical pulses via the electrode contacts in the A region at the same time and at the same place.

Although the conventional wisdom is to avoid providing simultaneous acoustic stimulation and electric stimulation to an A region, the inventor has determined that under the right conditions such simultaneous stimulation may be beneficial. This kind of simultaneous stimulation previously has been avoided because of the inherent stimulation mismatch arising from the different natures of stimulation types. For example, natural acoustic stimulation is much more frequency selective than artificial electric stimulation with state-of-the-art speech coding strategies. Nevertheless it has surprisingly turned out that such simultaneous stimulation may be beneficial for the patient if:
- There is a frequency match between the acoustic stimulation and the electrical stimulation. That is, the electrode contacts located in the A region are transferring information about the same sound frequencies as is the acoustic stimulation, where the match is as close as possible. An optimal or near-to-optimal electrical stimulation frequency may be derived from a post-operative CT scan to identify the exact location of the electrode contacts and adjust the stimulation frequencies on the contacts in the A region according to the Greenwood function (tonotopically); and
- complete fine structure information is present in the electrical stimulation. So if, for example, the A region is up to 500 Hz, then the fine structure is presented up to above 500 Hz for the assigned electrode channels.

Figure 5 is a flow chart showing various logical steps in producing coordinated electrical and acoustic stimulation signals in an electric acoustic stimulation (EAS) hearing system according to an embodiment of the present invention. A pseudo code example of such a method can be set forth as:

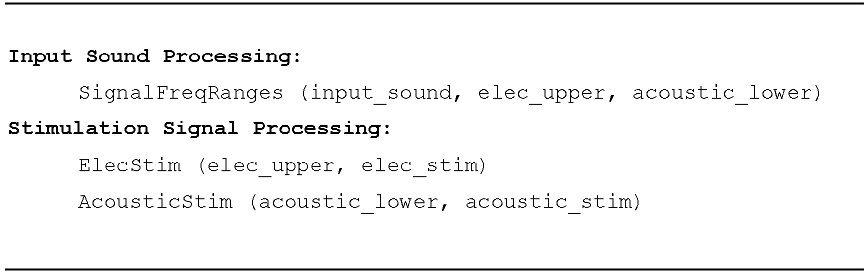

Figure 6 shows various functional blocks in a combined electrical and acoustic stimulation system according to an embodiment of the present invention. The details of such an arrangement are set forth in the following discussion.

As in the arrangement discussed above with respect to Figure 2, a preprocessor signal filter bank **601** can be configured to decompose an input sound signal into band pass frequency component signals *B*₁ to *B*_{M}, step **501,** representing an estimate of instantaneous input frequency/timing and component level/amplitude such that each band pass frequency component signal *B*₁ to *B*_{M} changes over time in characteristic timing and amplitude. The timing of the band pass frequency component signals *B*₁ to *B*_{M} typically may reflect frequency-specific response latencies and/or phase characteristics. The hearing signal processing module **602** then processes the band pass frequency component signals *B*₁ to *B_{M}* to generate electrical communications signals *S*₁ to *S*_{N} which are representative of an upper range of sound frequencies present in the input sound signal, and also an acoustic communications signal *S*_{A} which is representative of a lower range of sound frequencies present in the input sound signal.

An implanted electrical stimulation subsystem includes a pulse mapping module **603** and a pulse shaper **604** as described above with respect to Figure 2, which are configured to receive the electrical communications signals *S*₁ to *S*_{N} from the hearing signal processor **602** and deliver corresponding electrical stimulation signals *E*₁ to *E*_{M} to the electrode contacts for electrical stimulation of adjacent neural tissue, step **502.** One or more of the electrode contacts towards the apical end of the electrode array is located in an acoustically perceivable cochlear region which retains some residual natural hearing. An external acoustic stimulation subsystem **605** is configured to receive the acoustic communications signal *S*_{A} from the hearing signal processor **602** and deliver corresponding amplified acoustic stimulation signals *A* to the ear canal of the patient, step **503.** The hearing signal processor **602** further is configured to overlap the upper range and the lower range and coordinate the electrical stimulation signals *E*₁ to *E*_{N} and the amplified acoustic stimulation signals *A* for simultaneous delivery to the acoustically perceivable cochlear region.

As indicated above, the electrical stimulation signals *E*₁ to *E*_{M} in specific embodiments may be FSSS and/or CSSS stimulation signals. For example, the length of the FSSS can vary based on the number of electrode channels and the number of the CSSS per channel. The lengths of the electrode channel CSSS per FSSS may be constant, however, varying CSSS lengths per FSSS also may be possible, such as longer CSSS at more apical channels or longer/shorter CSSS at the maximum level of the FSSS, etc. Some embodiments also may apply a Channel Interaction Compensation (CIC) algorithm (e.g., U.S. Patent 7,917,224) to the amplitudes of simultaneous FSSS to provide a desired loudness level to the user. The onset of the CSSS within a FSSS is controlled by the phase of the travelling wave. Subthreshold stimulation on individual electrode channels may be applied within a single FSSS in order to support and maintain spontaneous action potentials at the stimulation locations. Frequency specific characteristics of the FSSS such as amplitude shape, spread over electrode positions, and duration (of entire FSSS and channel specific CSSS per FSSS) can be stored as templates in system memory that is accessible to the hearing signal processing module **602.**

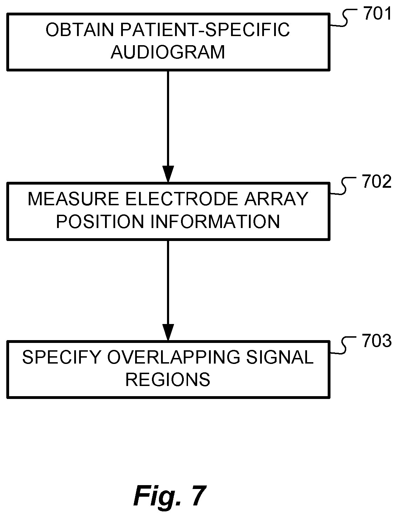

Figure 7 shows various logical blocks in a process of fitting a combined electrical and acoustic stimulation system according to an embodiment of the present invention. The first steps in such a fitting process would involve obtaining a patient audiogram, step **701,** and measuring information about the electrode position in the cochlea such as the electrode insertion angle, step **702.** Then the acoustically perceivable cochlear region is determined, step **703,** where one or more electrode contacts at the apical end of the inserted electrode array overlaps with the region of residual natural hearing; for example, by applying the Greenwood function to the electrode insertion angles. Then the electrode contacts in the acoustically perceivable cochlear region can be fit based on applying simultaneous electrical acoustic stimulation to those contacts. The remaining electrode contacts and the acoustic stimulation subsystem can be fit following known procedures. Rate-location matched stimulation may be applied on all channels independently of whether they are within the acoustic region or not.

Patients fitted as described above with simultaneous electrical and acoustic stimulation to an acoustically perceivable cochlear region may realize better sound perception in difficult conditions such as noise. In addition, the fitting procedure may actually take less time since there is no need to decide which electrode contacts have to be turned off. In addition, there is no need to change the electrical stimulation range when natural hearing changes (e.g. when the frequency range of natural hearing further decreases over time after implantation).

Embodiments of the invention may be implemented in part in any conventional computer programming language. For example, preferred embodiments may be implemented in a procedural programming language (*e.g*., "C") or an object oriented programming language (*e.g*., "C++", Python). Alternative embodiments of the invention may be implemented as pre-programmed hardware elements, other related components, or as a combination of hardware and software components.

Embodiments can be implemented in part as a computer program product for use with a computer system. Such implementation may include a series of computer instructions fixed either on a tangible medium, such as a computer readable medium (*e.g*., a diskette, CD-ROM, ROM, or fixed disk) or transmittable to a computer system, via a modem or other interface device, such as a communications adapter connected to a network over a medium. The medium may be either a tangible medium (*e.g*., optical or analog communications lines) or a medium implemented with wireless techniques (*e.g*., microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (*e.g*., shrink wrapped software), preloaded with a computer system (*e.g*., on system ROM or fixed disk), or distributed from a server or electronic bulletin board over the network (*e.g*., the Internet or World Wide Web). Of course, some embodiments of the invention may be implemented as a combination of both software (*e.g*., a computer program product) and hardware. Still other embodiments of the invention are implemented as entirely hardware, or entirely software (*e.g*., a computer program product).

Although various exemplary embodiments of the invention have been disclosed, it should be apparent to those skilled in the art that various changes and modifications can be made which will achieve some of the advantages of the invention without departing from the true scope of the invention.

## Claims

1. An electric acoustic stimulation (EAS) hearing system comprising:
a preprocessor signal filter bank (601) configured to decompose an input sound signal into a plurality of band pass frequency component signals (B₁ - B_{M}) that can be roughly characterized as a sinusoid at the center frequency of the band pass filter which is modulated by an envelope signal;
a hearing signal processor (602) configured to process the band pass signals (B₁ - B_{M}) and generate:
i. an electrical communications signal (S₁ - S_{M}) representative of an upper range of sound frequencies present in the input sound signal, and
ii. an acoustic communications signal (S_{A}) representative of a lower range of sound frequencies present in the input sound signal;
an implanted electrical stimulation subsystem including an electrode array having a plurality of electrode contacts implanted in a patient cochlea with one or more electrode contacts in an acoustically perceivable cochlear region retaining residual natural hearing, wherein the electrical stimulation subsystem is configured to receive the electrical communications signal (S₁ - S_{M}) from the hearing signal processor (602) and deliver corresponding electrical stimulation signals (E₁ - E_{M}) to the electrode contacts for electrical stimulation of adjacent neural tissue; and
an external acoustic stimulation subsystem (605) configured to receive the acoustic communications signal from the hearing signal processor and deliver corresponding amplified acoustic stimulation signals (A) to -an ear canal of the patient;
wherein the hearing signal processor (602) is configured to include fine structure information in the electrical stimulation signals (E₁ - E_{M}) for the one or more electrode
contacts; wherein the fine structure information is associated with said sinusoid; and
wherein the hearing signal processor (602) is configured to overlap the upper range and the lower range and coordinate the electrical stimulation signals (E₁ - E_{M}) and the amplified acoustic stimulation signals (A) for simultaneous delivery to the acoustically perceivable cochlear region such that there is a frequency match between the acoustic stimulation and the electrical stimulation.

2. The system according to claim 1, wherein the hearing signal processor (602) is configured to generate frequency specific sampling sequence (FSSS) signals as the electrical stimulation signals (E₁ - E_{M}).

3. The system according to claim 1, wherein the hearing signal processor (602) is configured to generate channel specific sampling sequence (CSSS) signals as the electrical stimulation signals (E₁ - E_{M}).

4. The system according to claim 1, wherein the hearing signal processor (602) is configured to generate electrical stimulation signals (E₁ - E_{M}) as a patient-specific, frequency-specific function of stimulation location, rate, and level.

5. The system according to claim 1, wherein the one or more electrode contacts in the acoustically perceivable cochlear region are implanted at least 24 mm into the patient cochlea.

## Patentansprüche

1. Hörsystem für elektrisch-akustische Stimulation (EAS), umfassend:
eine Vorprozessorsignalfilterbank (601), die konfiguriert ist, um ein Eingangsschallsignal in eine Vielzahl von Bandpassfrequenzkomponentensignalen (B₁ - BM) zu zerlegen,
die als ein Sinussignal bei der Mittenfrequenz des Bandpassfilters charakterisiert werden können, das durch ein Hüllkurvensignal moduliert wird;
einen Hörsignalprozessor (602), der konfiguriert ist, um die Bandpasssignale (B₁ - BM) zu verarbeiten und folgendes zu erzeugen:
i. ein elektrisches Kommunikationssignal (S₁ - S_{M}), das für einen oberen Bereich von Schallfrequenzen repräsentativ ist, die in dem Eingangsschallsignal vorhanden sind, und
ii. ein akustisches Kommunikationssignal (S_{A}), das für einen unteren Bereich von Schallfrequenzen repräsentativ ist, die in dem Eingangsschallsignal vorhanden sind;
ein implantiertes elektrisches Stimulationssubsystem, das eine Elektrodenanordnung mit einer Vielzahl von Elektrodenkontakten umfasst, die in eine Patientencochlea implantiert sind, wobei ein oder mehrere Elektrodenkontakte sich in einer akustisch wahrnehmungsfähigen Cochlearegion befinden, die restliches natürliches Gehör beibehält, wobei das elektrische Stimulationssubsystem konfiguriert ist, um das elektrische Kommunikationssignal (S₁ - S_{M}) von dem Hörsignalprozessor (602) zu empfangen und entsprechende elektrische Stimulationssignale (E₁ - E_{M}) an die Elektrodenkontakte zur elektrischen Stimulation von benachbartem Nervengewebe zu liefern; und
ein externes akustisches Stimulationssubsystem (605), das dazu konfiguriert ist, das akustische Kommunikationssignal von dem Hörsignalprozessor zu empfangen und entsprechende verstärkte akustische Stimulationssignale (A) an einen Gehörgang des Patienten zu liefern;
wobei der Hörsignalprozessor (602) dazu konfiguriert ist, Feinstrukturinformationen in die elektrischen Stimulationssignale (E₁ - E_{M}) für den einen oder die mehreren Elektrodenkontakte aufzunehmen;
wobei die Feinstrukturinformationen mit dem Sinussignal assoziiert sind und
wobei der Hörsignalprozessor (602) dazu konfiguriert ist, den oberen Bereich und den unteren Bereich zu überlappen und die elektrischen Stimulationssignale (E₁ - E_{M}) und die verstärkten akustischen Stimulationssignale (A) zur gleichzeitigen Lieferung an die akustisch wahrnehmungsfähige Cochlearegion zu koordinieren, so dass es eine Frequenzübereinstimmung zwischen der akustischen Stimulation und der elektrischen Stimulation gibt.

2. System nach Anspruch 1, wobei der Hörsignalprozessor (602) dazu konfiguriert ist, frequenzspezifische Abtastsequenzsignale (FSSS-Signale) als die elektrischen Stimulationssignale (E₁ - E_{M}) zu erzeugen.

3. System nach Anspruch 1, wobei der Hörsignalprozessor (602) dazu konfiguriert ist, kanalspezifische Abtastsequenzsignale (CSSS-Signale) als die elektrischen Stimulationssignale (E₁ - E_{M}) zu erzeugen.

4. System nach Anspruch 1, wobei der Hörsignalprozessor (602) dazu konfiguriert ist, elektrische Stimulationssignale (E₁ - E_{M}) als eine patientenspezifische, frequenzspezifische Funktion von Stimulationsort, -rate und -pegel zu erzeugen.

5. System nach Anspruch 1, wobei der eine oder die mehreren Elektrodenkontakte in der akustisch wahrnehmungsfähigen Cochlearegion mindestens 24 mm in die Patientcochlea implantiert sind.

## Revendications

1. Un système auditif à stimulation électro-acoustique (EAS) comprenant :
une banque de filtres de signaux de prétraitement (601) qui est configurée pour décomposer un signal sonore d'entréeen une pluralité de signaux de composante fréquentielle passe-bande (B1 - BM) qui peuvent être grossièrement caractérisés comme une sinusoïde à la fréquence centrale du filtre passe-bande qui est modulée par un signal d'enveloppe ;
un processeur de signal auditif (602) qui est configuré pour traiter les signaux passe-bande (B1 - BM) et générer :
i. un signal de communication électrique (S1 - SM) représentatif d'une plage supérieure
de fréquences sonores présentes dans le signal sonore d'entrée, et
ii. un signal de communication acoustique (SA) représentatif d'une plage inférieurede fréquences sonores présentes dans le signal sonore d'entrée ;
un sous-système de stimulation électrique implanté comprenant un réseau d'électrodes ayant une pluralité de contacts d'électrode implantés dans une cochlée du patient avec un ou plusieurs contacts d'électrode dans une région cochléaire perceptible acoustiquement qui conserve une audition naturelle résiduelle,
dans lequel le sous-systèm e de stimulation électrique est configuré pour recevoir le signal de communication électrique (S1 - SM) du processeur de signal auditif (602) et délivrer des signaux de stimulation électrique correspondants (E1 - EM) aux contacts d'électrode pour la stimulation électrique du tissu neural adjacent ; et
un sous-système de stimulation acoustique externe (605) qui est configuré pour recevoir le signal de communication acoustique du processeur de signal auditif et délivrer des signaux de stimulation acoustique amplifiés correspondants (A) à un conduit auditif du patient ;
dans lequel le processeur de signal auditif (602) est configuré pour inclure une information de structure fine dans les signaux de stimulation électrique (E1 - EM) pour le ou les contacts d'électrode ; dans lequel l'information de structure fine est associée à ladite sinusoïde ;
et
dans lequel le processeur de signal auditif (602) est configuré pour faire chevaucher la plage supérieure et la plage inférieure et coordonner les signaux de stimulation électrique (E1 - EM) et les signaux de stimulation acoustique amplifiés (A) pour une délivrance simultanée à la région cochléaire perceptible acoustiquement de sorte qu'il existe une correspondance de fréquence entre la stimulation acoustique et la stimulation électrique.

2. Le système selon la revendication 1, dans lequel le processeur de signal auditif (602) est configuré pour générer des signaux de séquence d'échantillonnage spécifique à la fréquence (FSSS) comme signaux de stimulation électrique (E1 - EM).

3. Le système selon la revendication 1, dans lequel le processeur de signal auditif (602) est configuré pour générer des signaux de séquence d'échantillonnage spécifique au canal (CSSS) comme signaux de stimulation électrique (E1 - EM).

4. Le système selon la revendication 1, dans lequel le processeur de signal auditif (602) est configuré pour générer des signaux de stimulation électrique (E1 - EM) comme une fonction spécifique au patient et spécifique à la fréquence de l'emplacement de stimulation, du taux de stimulation et du niveau de stimulation.

5. Le système selon la revendication 1, dans lequel le ou les contacts d'électrode dans la région cochléaire perceptible acoustiquement sont implantés à au moins 24 mm dans la cochlée du patient.
